# EUROPEAN PATENT APPLICATION

(11) **EP 4 327 754 A2**
(43) Date of publication of application: **28.02.2024**
(21) Application number: 23192830.0
(22) Date of filing: 22.08.2023
(51) Int. Cl.: A61B 10/00

(54) **SAMPLING CAPSULE SYSTEM AND METHODS**

(30) Priority: 27.08.2022 US 202263401624 P; 31.08.2022 US 202263402673 P; 14.01.2023 US 202363439094 P; 15.08.2023 US 202318234063
(71) Applicant: Jones, James Phillip, Sudan, TX 79371 (US)
(72) Inventor: Jones, James Phillip, Sudan, TX 79371 (US)
(74) Representative: Script IP Limited

(57) **Abstract**

A non-invasive system for sampling gastrointestinal microbiota with their associated environment for discovery, characterization, medical research, diagnostics, and treatment using an ingestible, non-digestible sampling capsule. The capsule device, with ports open for acquiring a sample of gastrointestinal microbiota and content, is placed inside an immediate or delayed release capsule. When the outer capsule dissolves according to its specifications, enteric fluid and content enters the sampling capsule through the ports triggering a hydrophilic stop to release a pulling force to close the ports by enveloping the capsule outer casing over the inner casing, capturing the sample, and holding it sealed until it completes passage through the digestive track, and is recovered from feces for analysis.

## Description

### PRIORITY APPLICATIONS

The present application claims priority from each of U.S. Provisional Patent Applications 63/401,624 filed on August 27, 2022; 63/402,673 filed on August 31. 2022; and 63/439,094 filed on January 14, 2023; and U.S. Utility Patent Application 18/234,063 filed on August 15, 2023.

### BACKGROUND OF THE INVENTION

### Field of Invention

The present invention relates to at least one device, at least one system, and at least one process or method to be used within the medical sciences, engineering, research and medical technologies. The device is ingestible, untethered, and is designed to collect samples from, or release substances into, a gastrointestinal tract.

It is to be understood that embodiments described herein are not intended to be limited in their application to the details of construction and the arrangement of the components set forth in the following description. The subject matter of the invention(s) is capable of other embodiments or of being practiced or carried out in various ways. Also, it is to be understood that the phraseology and terminology employed herein is for the purpose of description and should not be regarded as limiting.

### Description of the Related Art

Major scientific research and medical efforts are being dedicated to discovering an ever-increasing amount of knowledge regarding the roles played in human and animal life and disease by gastrointestinal microbes. An individual's gastrointestinal microbiome varies with location and time due to a multitude of factors including symbiosis and antibiosis relationships, time, ingesta, age, and health. Microbiota taxa vary from location to location in the digestive tract becoming increasingly diverse and abundant through the differing stages of the digestive tract from the stomach to the colon. One published estimate indicates that approximately 10^4 microbes are found in the small intestine and 10^10 microbes (or 50% of the fecal mass) are found in the colon. The nature of the microbes and their function remain much of a mystery due to inaccessibility of the gut, the lack of an effective means to sample and characterize them, and the complex symbiotic relationships in their localized taxa.

Medicine has advanced in all major human body systems, such as cardiovascular, neurological, muscular and skeletal, but the intestinal tract still remains much of a mystery. Though there are instruments and devices which can be used for endoscopy/colonoscopy, there are sections of intestines that remain unexplored and are found incredibly difficult to collect and preserve samples there from. Even these upper and lower extremities, which can be viewed by camera and can only be treated for visible damage, such as polyps or ulcers. One known device is a camera pill that can now be swallowed and pictures taken throughout the intestinal tract. But visible inspection does not address the scientific mysteries of diseases and their causes or cures.

The human gut represents a void in medical science. The roles that bacteria play in the intestinal tract are poorly understood, except that there are "good" and "bad" bacteria. In fact, only a few of the estimated thousands of strains of bacteria are known, or have been identified, have been characterized and their roles determined. An aim of the subject matter disclosed herein is to help answer questions such as: what biochemical products exist for any specific diet as a function of the gut length x; what biochemical reactions take place along the gut length x; what microbes exist at any point within the gut anatomical system; what are the byproducts of all microbes including their toxins, exotoxins and endotoxins, and virulence factors and enzymes existing within the gut.

Further, the interaction of a) the normal biochemical reactions, b) the microbes, and c) the microbe byproducts are largely unknown, and believed to be a major source of several major diseases. Some 15 categories of bacteria have been broadly identified within the Phylogenic Tree as existing within major components of the gastrointestinal tract. Thus, in general, bacteria strains and colonies and their populations, population densities, habitats, and characteristics and contributions to the digestive process are only vaguely known in healthy individuals, and largely unknown in unhealthy individuals, much less as a function of gut length x.

The inaccessible regions of the most important anatomy of the gut, the lack of technology to explore, discover, and experiment in an in vivo manner, and then administer medications and measure in vivo the immediate results, has constrained the advance of medical science pertaining to the gut. A competent research effort investigating any animate or inanimate system should attempt to identify the fundamental multidisciplinary scientific principles of science and engineering upon which the system is based and functions, and then develop quantitative measures of those principles. The inaccessibility of the gut, and the heretofore lack of exploratory technology, has resulted in speculation and statistical correlation of symptoms from a distance throughout history as a means of researching the gut. The need for in vivo technology became immediately apparent. The first step to further this technology is the determination of what exists at various distances x along the gastrointestinal tract.

Non-invasive sampling processes are presently limited to oral spit, internal swabs, and feces analysis. Minimal invasive sampling processes include swabs of the rectum. They do not provide for examination of localized microbiota community content within the gut that is important to gain understanding of bacteria and host-bacterial interactions. This problem is complicated by changing environmental conditions such as pH, the release of resident bacteria and other microbiota (alive and dead), body chemistry, particles, and fluids from upstream regions of the gastrointestinal tract that transit through the intestines and are subsequently detectable in feces. The intraluminal pH rapidly changes from highly acidic in the stomach (pH 1-3.5) to about pH 6 in the duodenum. The pH gradually increases in the small intestine from pH 6 to about pH 7.4 in the terminal ileum. The pH drops to 5.7 in the caecum, but again gradually increases, reaching pH 6.7 in the rectum. Feces confounds characterization of microbial communities that are extremely diverse and include a large number of Gram-positive eubacteria, Gram- negative bacteria, cyanobacteria, archaea, fungi, protozoa, and virus as luminal communities within the colon that differ, for example, from dynamic mucosa-associated communities found in the small intestine.

Invasive gastroscopy and endoscopy procedures are used to sample microbiota of the upper gastrointestinal track of the esophagus, stomach, and duodenum and for the lower intestinal tract of the colon, large intestine, and lower portion of small intestine, but approximately 5m of ileum and jejunum cannot be reached for scope sampling. Both scopes necessitate expensive procedures and require anesthesia, risk, and recovery in a medical setting. They are a poor strategy for time course data acquisition, diagnosis support, or treatment support.

Efforts to find an effective method to sample intestinal microbiota in their local areas have not been successful. Indeed, citations show there have been inventive efforts including ingestible capsules operated with electrical circuits, batteries, radio, magnetic fields, steel compression and torsion springs working alone or together, and vacuums. Examples of such contributions may be found in U.S. Pat. Nos. 8,491,495, 8,915,863, 8,926,526, 9,215,997, and 9,955,922 to Shuck and all of which are included herein by reference in their entireties. However, these devices and methods are not in present use and appear to be impractical for production or general use. Research, medical communities, and patients beg novel solutions that can be repetitively used at home or in a general medical practice setting.

The subject matter herein comprises mechanical devices, systems, and methods for acquiring samples of matter along an intestinal track of a user in-vivo. The device is a capsulized device and system configured to be swallowed and passed through the intestinal track. The device comprises a hollow casing or housing defining one or more openings adapted to allow samples of liquid and semi-liquid matter to pass into the housing during a specified time interval and then sealed therein for collection outside the body for analysis. Further, some embodiments are capable of releasing substances stored within the device into the surrounding environment.

### SUMMARY

The subject matter presented herein describes a sampling system comprising an inner capsule and an outer casing. The inner casing comprises a ported first casing with an open end, a closed end, an inner surface, and an outer surface and a ported second casing with an open end, a closed end, an inner surface, and an outer surface. The system further comprises a hydrophobic, acid resistant elastomeric filament fixedly attached to the closed end of the ported first casing and to the closed end of the ported second casing, a hydrophilic strut, wherein the hydrophilic strut spaces the closed end of the ported first casing away from the closed end of the ported second casing and is of such a length as to impart a tension in the hydrophobic elastomeric filament; and a first digestible outer capsule enveloping the inner capsule, the first digestible outer capsule being configured to temporarily isolate the inner capsule from bodily fluids.

The subject matter presented herein also describes a gastrointestinal sampling device comprising a hollow capsule having a ported inner casing with a closed end and an open end and having a ported outer casing with a closed end and an open end, an elastomeric filament fixedly securing the closed end of the inner casing to the closed end of the outer casing; and a hydrophilic strut spacing the closed end of the inner casing away from the closed end of the outer casing when intact.

The subject matter presented here in further describes a gastrointestinal sampling system comprising a hollow capsule having a ported inner casing having a closed end and an open end slidingly engaged within a ported outer casing also having a closed end and an open end; and, an elastomeric filament fixedly securing the closed end of the inner casing to the closed end of the outer casing.

### BRIEF DESCRIPTIONS OF THE DRAWINGS

FIG. 1 depicts a perspective view of an exemplary ported inner casing of the gastrointestinal sampling device according to an embodiment.
FIG. 2 depicts a perspective view of an exemplary ported outer casing of the gastrointestinal sampling device according to an embodiment.
FIG. 3 depicts a cross-sectional view of an exemplary ported inner casing of the gastrointestinal sampling device according to an embodiment.
FIG. 4 depicts a cross-sectional view of an exemplary ported outer casing of the gastrointestinal sampling device according to an embodiment.
FIG. 5 depicts a perspective view of an assembled gastrointestinal sampling device in the strutted, open configuration.
FIG. 6 depicts a perspective view of the gastrointestinal sampling device in the closed configuration.
FIG. 7 is a side view of the gastrointestinal sampling device in the strutted open configuration.
FIG. 8 is a cross sectional view of the gastrointestinal sampling device of FIG. 7.
FIG. 9 is a side view of the gastrointestinal sampling device of FIG. 7 in the sealed closed configuration after the hydrophilic elastomeric filament fails.
FIG. 10 depicts an exploded perspective view of a gastrointestinal sampling device according to an embodiment with a compound strut.
FIG. 11 is a cross-sectional view of the outer ported casing of FIG. 10.
FIG. 12 is a perspective view of the assembled gastrointestinal sampling device of FIG. 10 in its open configuration.
FIG. 13 is a perspective view of the assembled gastrointestinal sampling device of FIG. 10 in its closed configuration.
FIG. 14 is a cross sectional view of an assembled gastrointestinal sampling system in its open configuration inside a digestible outer casing.
FIG. 15 is a cross sectional view of an assembled gastrointestinal provision system in its closed configuration inside a digestible outer casing.
FIG. 16 is a cross sectional view of an assembled gastrointestinal provision system in its closed configuration inside a digestible outer casing and an inner thin casing.
FIG. 17 is a depiction of a strut comprising an extended spring held in an elongated position by a hydrophilic substance.
FIG. 18 is a side view of a collapsible strut or strut clip embodiment combined with a spring.
FIG. 19 is a perspective view of a collapsible strut or strut clip embodiment combined with a spring.
FIG. 20 is an equivalent alternative embodiment of an ingestible capsule including a free form or unsecured absorbent material, a gasket, and a free form or unsecured embodiment of a destructible strut.
FIG. 21 is a block flow diagram of a sample capsule operating method.
FIG. 22 is an equivalent alternative exemplary embodiment comprising a preservative blister, bubble, or pack in an open configuration.
FIG. 23 is an equivalent alternative exemplary embodiment comprising a preservative blister, bubble, or pack in a collapsed configuration.
FIG. 24 is an equivalent alternative embodiment comprising a linear generator.
FIG. 25 is a drawing of an equivalent alternative embodiment wherein an external insert takes the place of a strut.
FIG. 26 is a presentation equivalent alternative exemplary embodiment utilizing an elastomeric loop and an insert.
FIG. 27 is a depiction of an equivalent embodiment with an external elastomeric filament.
FIG. 28 is a flow block diagram of a method for constructing at least one embodiment disclosed herein.
FIG. 29 is a depiction of a partially manufactured capsule according to the manufacturing method of FIG. 28.
FIG. 30 is a perspective view of an equivalent embodiment of capsule with a piggyback casing.

### DETAILED DESCRIPTION

The following detailed description is merely exemplary in nature and is not intended to limit the invention, or the application, or uses of the subject matter disclosed. As used herein, the word "exemplary" means "serving as an example, instance, or illustration." Thus, any embodiment described herein as "exemplary" is not necessarily to be construed as preferred or advantageous over other embodiments. All of the embodiments described herein are exemplary unpowered embodiments provided to enable persons skilled in the art to make or use the invention and not to limit the scope of the invention which is defined by the claims. As used herein, the term "unpowered" means "without a battery." Furthermore, there is no intention to be bound by any expressed or implied theory presented in the preceding technical field, background, brief summary, or the following detailed description.

The terminology used herein is for the purpose of describing particular embodiments only and is not intended to be limiting of the invention. As used herein, the term "and/or" includes any and all combinations of one or more of the associated listed items. As used herein, the singular forms "a," "an," and "the" are intended to include the plural forms as well as the singular forms, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof.

It should be understood that the terms "right" and "left" are used solely to denote opposite sides of an element, component, or surface in the same manner that "top" and "bottom" are used solely to denote opposite sides of an element, component, or surface and should not unnecessarily be construed as limiting the position or orientation of said element, component, or surface.

In the following description, for purposes of explanation, numerous specific details are set forth in order to provide a thorough understanding of the present invention. It will be evident, however, to one skilled in the art that the present invention may be practiced without these specific details.

In this document, relational terms such as first and second, and the like may be used solely to distinguish one entity or action from another entity or action without necessarily requiring or implying any actual such relationship or order between such entities or actions. Numerical ordinals such as "first," "second," "third," etc. simply denote different singles of a plurality and do not imply any order or sequence unless specifically defined by the claim language. The sequence of the text in any of the claims does not imply that process steps must be performed in a temporal or logical order according to such sequence unless it is specifically defined by the language of the claim. The process steps may be interchanged in any order without departing from the scope of the invention as long as such an interchange does not contradict the claim language and is not logically nonsensical.

Furthermore, depending on the context, words such as "connect" or "coupled to" used in describing a relationship between different elements do not imply that a direct physical connection must be made between these elements. For example, two elements may be connected to each other physically, electronically, logically, or in any other manner, through one or more additional elements.

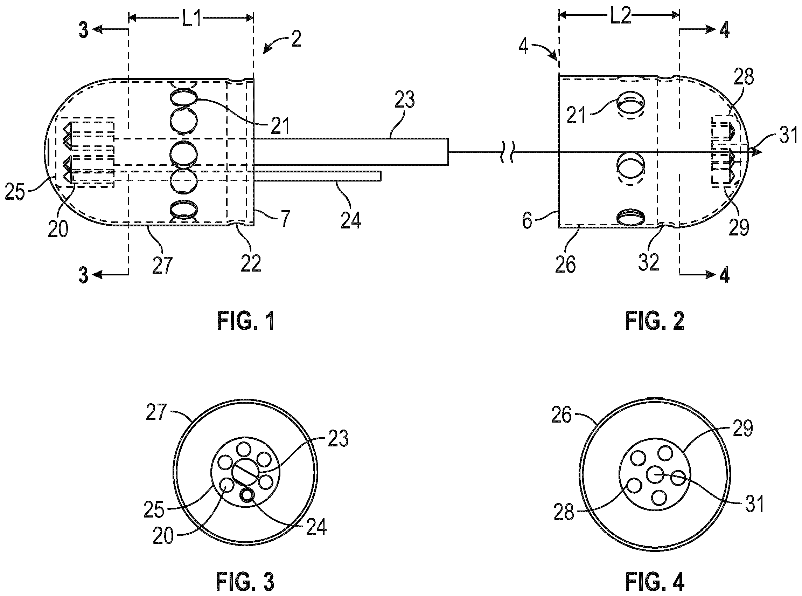

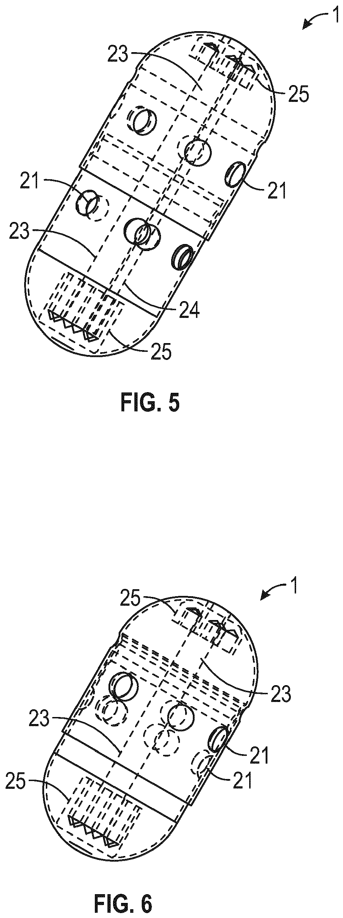

Reference should now be made to the drawings in which the same reference numbers are used throughout the various figures to designate the same or similar components. **FIG. 1** (inner casing **2**) and **FIG. 2** (outer casing **4**) are each one-piece acid tolerant medicinal grade elastomer or metallic devices with ports or holes **21** that penetrate through casing walls **27** and **26**, respectively. When assembled, the inner casing **2** and the outer casing **4** comprise the gastrointestinal sampling device **1** (**See,** **FIG. 5**). The inner casing **2** and the outer casing **4** may be circular cylinders, each with a domed closed end portion to ease transit though the digestion system. Each casing may also be referred to as a "half-capsule." However, different shapes may be employed that provide efficient or structural advantage in a given situation, including a simple sphere. As other non-limited examples, the outer casing may be formed as an ogive ended cylinder or an ogive ended cylinder with an irregular distal end. In this instance, an "irregular end" may manifest itself as The inner casing **2** or outer casing **4** or both may be constructed, at least partially, of a ferrous metal such that one could facilitate locating and extracting the sample capsule from a stool. Including a magnet in a polymer capsule would also be effective as an alternative in this regard.

As a non-limiting example, a gastrointestinal sampling device **1** may have an open configuration length of approximately 24 mm and a closed configuration length of approximately 20 mm. The inner casing **2** may have a diameter of approximately 9.0 mm. The outer casing **4** may have a diameter of approximately 9.91 mm. When in the closed configuration, the volume of the enclosed sample may be approximately 0.50 ml or eight (8) drops of fluid sample. The material of the gastrointestinal sampling device **1** may be a molded biomedical ingestible grade elastomer with a Shore A hardness of approximately 40.

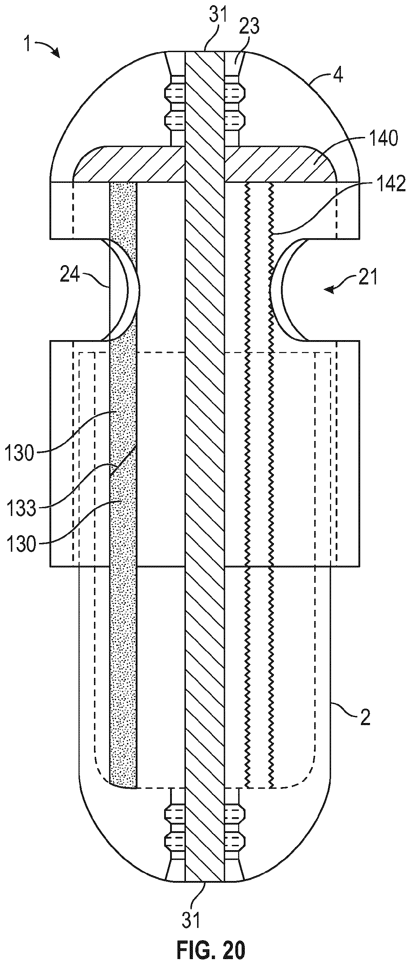

The number of ports **21**, their size, shape, or their configuration on the casings may be varied to satisfy sample collection or material delivery efficacy. However, it should be noted that the casing walls **26** and **27** are of such lengths **L1** and **L2** so as to cover and effectively seal the ports **21** in the other casing's walls such that all of the ports **21** in the gastrointestinal sampling capsule are fluid tight to ensure that the sample enclosed therein does not leak out after the time of closing. In some equivalent embodiments either the inner casing **2** or the outer casing **4** may have no ports **21** (See, e.g., **FIG. 20**).

Each of the inner casing **2** and outer casing **4** may include its own receiver **25** and **29**, respectively. Receivers **25** and **29** may be used to guide and support a strut **24** within one pair of multiple channels **20** and **28**, respectively.

In some embodiments, strut **24** may be physically fashioned out of a single piece of medical grade hydrophilic material such that the hydrophilic action strut **24** rapidly fails upon contact with a specified bowel material, acid, chemical or with fluids generally. Alternatively, the strut **24** may be engineered to fail after a period of time (a delayed action strut) rather than immediately (an immediate action strut). The time to fail may be controlled by the choice of material, the physical shape of the strut or both. One such material may be cellulose. Other possible non-limiting materials include polyvinyl alcohol, seaweed derivatives from the Evoware Company of Indonesia, casein proteins developed by the US Department of Agriculture, Agar, and the root of the cassava plant produced by the Avani Company of Bali. In short, any suitable medical grade dissolvable/digestible material may be used that currently exists or that may be developed in the future. The term "digestible" as used herein in regard to an outer capsule means "able to be softened or decomposed by heat and moisture or chemicals," such as may be found in the digestive tract. The term "ingestible" means "swallowable."

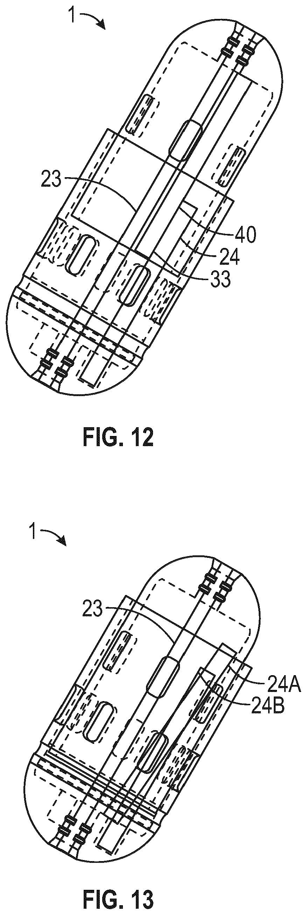

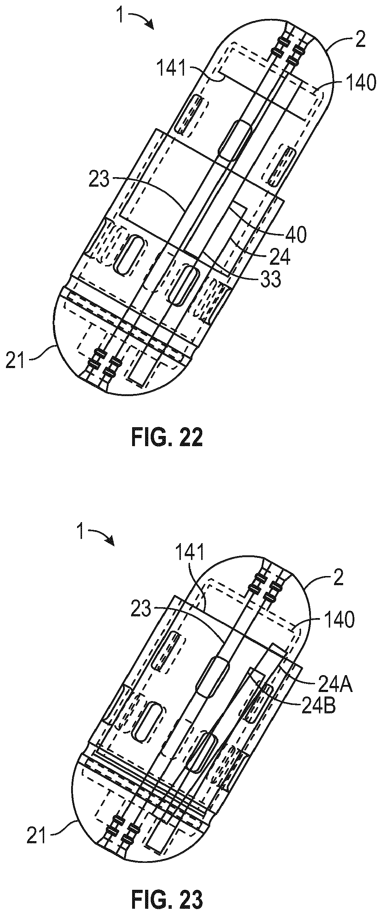

In other embodiments (See, e.g., **FIGs. 12-13**), strut **24** may be comprised of two or more hydrophobic members (**24A-B**) temporally connected by an attachment means **40** such as an adhesive, a pin, or similar means that may currently exist or be developed in the future. In some embodiments the attachment means may be a hydrophilic attachment means such that bowel material or fluid attacks only the attachment means **40** and not the hydrophobic members themselves that are allowed to subsequently detach. This multiple piece embodiment of strut **24** may also be referred to a hydrophilic action strut **24** but may be claimed in alternative language such as an immediate release strut. A hydrophilic action strut **24** may be configured such that each deconstructed hydrophobic half portion segment of the strut **24** are short enough to allow the gastrointestinal sampling device **1** to contract into its closed configuration, but long enough to extend into the closed ends of the casings **2** and **4** and pierce or impinge on the one or more bubbles, blisters, or sacks **140** containing the preservative, if any. The bubbles, blisters or sacks **140** may be unitary such that they can be inserted during assembly, or may be comprised of a dedicated section of casing 2, 4 isolated by a diaphragm that is filled from the outside during assembly (See, e.g., **FIGs. 22-23**).

In some equivalent embodiments, the hydrophobic half portions of the strut **24** may push against one or more membranes or diaphragms **141** that separate the preservative from the sample. The preservative **140** may be exuded through a pressure relief valve Z (not shown) in a diaphragm **Y** (not shown) of the one or more casings. The pressure relief valve may be as simple as a flap of material A (not shown) covering hole B (not shown) through, or bypassing, a diaphragm Y (not shown).

Similarly, a substance may be exuded via a relief valve through the casing into the bowel (not shown). One substance (e.g., medicine) may be exuded into the bowel through the wall of the inside casing and a second, different material (e.g., nano-objects) through the wall of the outer casing. The pressure relief valve may be any suitable device that will open and relieve over pressure below a diaphragm but may be as simple as a flap of material covering a hole through, or bypassing, a diaphragm **141** or a casing wall.

In other embodiments the attachment means **40** may be comprised of a pH sensitive material and may be termed a pH attachment means or a delayed action connection means. A non-limiting example of an adhesive suitable for the purpose of attaching two hydrophobic struts is polyvinyl alcohol (PVOH), which may be both a hydrophilic attachment means and a pH attachment means.

As a non-limiting example the strut **24** has a length **L** that may be approximately 20.5 mm and a diameter of 1.3 mm. Strut **24** may be a cylinder or a parallelepiped and may be further shaped to tailor the timing of its failure to a desired time period, including immediate failure upon contact with an intestinal fluid.

In some embodiments, inner casing **2** and outer casing **4** may be mated by inserting one end of strut **24** into one of the channels **(20, 28)** in receiver **(25, 29)** of one casing **(2, 4)** and the other end into a corresponding channel **(20, 28).** The number of channels **(20, 28)** may vary but there may be at least one channel in one of the two casings. However, strut 24 may be inserted into the capsule loose without insertion into a channel to facilitate assembly without adverse operational effect.

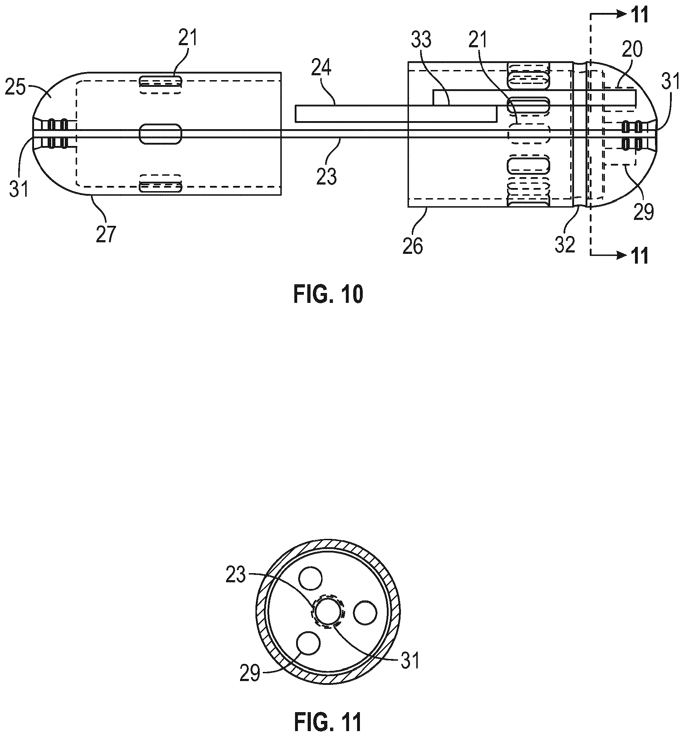

In some embodiments, the closed ends of the inner casing **2** and the outer casing **4** may each include a hole **31** penetrating through both of the center of the receiver and the center of closed end of each casing to accept and pass through an elastomeric filament **23**. (See, e.g.**,** **FIG 11**).

Elastomeric filament **23** may be attached to the same receivers **(25, 29)** as the strut **24** in both the inner and outer casings and is partially relaxed until both ends of strut **24** engage one of the channels **(20, 28)** in a casing, thereby requiring elastomeric filament **23** to stretch. In some embodiments the elastomeric filament **23** extends through the receiver(s) **(25, 29)** and into a hole **31** in the closed end(s) of one or both casings **(2, 4)** and is attached therein. The end of elastomeric filament **23** may be tensioned and then affixed to the walls of the receiver defining the hole **31** by welding or gluing to make the hole(s) **31** fluid tight. A non-limiting example of a glue for this purpose is Cyanoacrylate or Loctite HY 4070 manufactured by Henkel Adhesives. A non-limiting example of elastomeric filament **23** may be comprised of Elastostar R 401/40 from Elastostar Rubber Company of Columbus Ohio, which may be tensioned to 80% of its elastic limit in this application.

In other equivalent embodiments, channels (**20, 28**) may be dispensed with and the strut **24** may be configured of such a length that merely loosely placing the strut **24** in the capsule serves to engage the closed ends of the inner and outer casings, space the inner casing and the outer casing apart, and tension the elastomeric filament **23**.

When the inner casing **2** is partially inserted into the outer casing **4** and spaced apart by the strut **24** with a length **L,** the elastomeric filament **23** may be trimmed flush to the outer surface of casing **(2, 4)**. The length **L** of strut **24** is such that when inserted it maintains the gastrointestinal sampling device **1** in its open configuration with all ports **21** unobstructed while imparting a tension in the elastomeric filament **23** (see**,** **FIG. 5**). The tension strains against the strut to place the gastrointestinal sampling device **1** into its closed configuration (see, **FIG. 6**) when the strut collapses. It should be noted that the elastomeric filament **23** is of a relaxed length that is less than the distance between receivers **(25, 29)** in a closed configuration such that it continues to maintain the inner casing **2** and outer casing **4** into the closed configuration in the absence of an intact strut **24**.

In some embodiments the elastomeric filament **23** is comprised of an elastomer that is non-reactive to, or it is at least resistant to digestion by, bodily fluids. In some cases, the elastomeric filament **23** may be notably acid resistant if it is to operate in the stomach where the gastric environment is at a pH of 3.0 and lower. A non-limiting example of a substance that is suitable for the elastomeric filament **23** is a medical grade silicone rubber, a non-limiting example of which is Elastostar R 401/40.

Strut **24** holds the assembled gastrointestinal sampling device **1** in its open configuration, or open port position, against the pulling tension provided by the elastomeric filament **23** until the strut **24** is weakened by inflowing enteric fluids and collapses thereby allowing the pulling tension to further encourage travel of outer casing **4** over inner casing **2** to close ports **21** by overlap of the casings. The two casings are secured in the closed configuration by a male locking stop ring **32** in one of the casings that is configured to lock by interference fit into the female locking stop ring **22** in the other casing. The residual tension remaining in elastomeric filament **23** ensures that the gastrointestinal sampling device **1**, and the capsule ports **21,** remain closed while the gastrointestinal sampling device **1** and it captured sample is recovered. Instead, or in addition to, an O-ring may also be employed to help seal the device in its closed configuration.

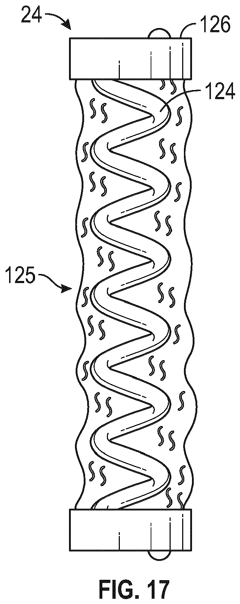

In other equivalent embodiments the strut **24** may be replaced with a spring **124** in an extended stressed condition (See **FIG. 17**). The extended condition of spring **124** may be maintained by encasing the stressed spring in a medical grade hydrophilic immediate or delayed release material such as PVOH **125**. When the PVOH is eroded away by the contact of bodily fluids, the spring is then able to contract to close the capsule **1**. Both ends of extended spring include mounting plates **126** that attach the extended spring **124** to each of the inner casing and the outer casing.

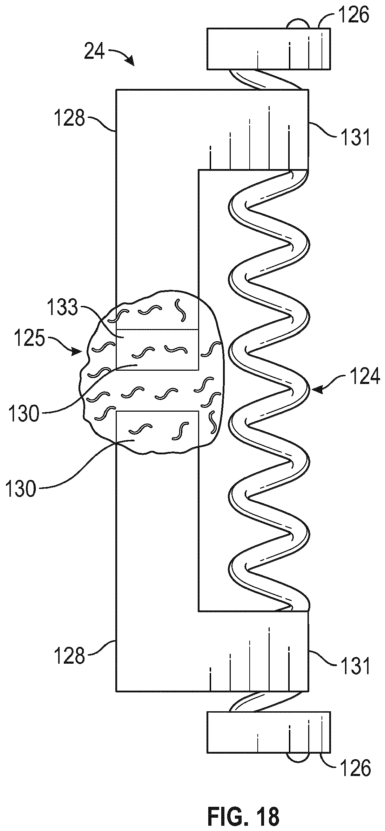

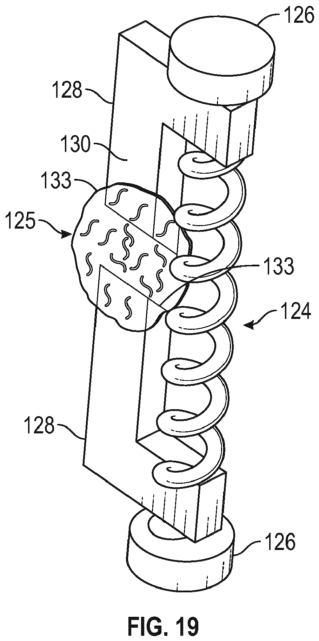

In still other embodiments (See **FIGs. 18-19**) the Strut **24** may be a bracket comprising an upper and lower L-shaped strut clip **128**. The strut clips may or may not be hydrophilic. The strut clips **128** may be joined together at their proximal ends **130**. Their respective distal ends **131** are coupled to the ends of the extendedly stressed spring **124** or to the mounting plates **126**, if used. The proximal ends **130** may be joined mechanically offset, or they may be beveled **133,** so that when the hydrophilic material deteriorates, the bracket collapses without binding, thereby allowing the spring **124** to retract to its relaxed position and close the capsule. In other embodiments the upper and lower L-shaped strut clips **128** may be joined mechanically with a hydrophobic material where the joint is then disrupted by electro-mechanical means, or by sound frequency signals.

In some embodiments the gastrointestinal sampling device **1** can include delayed release compounds to "fix" or preserve the sample flowing in through the ports. For example, the fluid flowing into the inner capsule triggers the strut collapse. At the same time, the fluid also starts dissolving the sample preservative. When the capsule is recovered it can be kept inside the closed capsule for storage. There are several chemical approaches to preserving samples as may be known in the art, but they should not have reactive effect on the microbiota. Such, a preservative may be obtained from DNA Genoteck, a subsidiary of Orasure Technologies located in Ottawa, Canada.

**FIGs. 3** and **4** are cross sectional views of **FIGs. 1** and **2**. As can be seen in **FIG. 3**, strut **24** is inserted in one of the chambers **20** of receiver **25**, and elastomeric filament **23** is secured to the closed end of inner casing **2.**

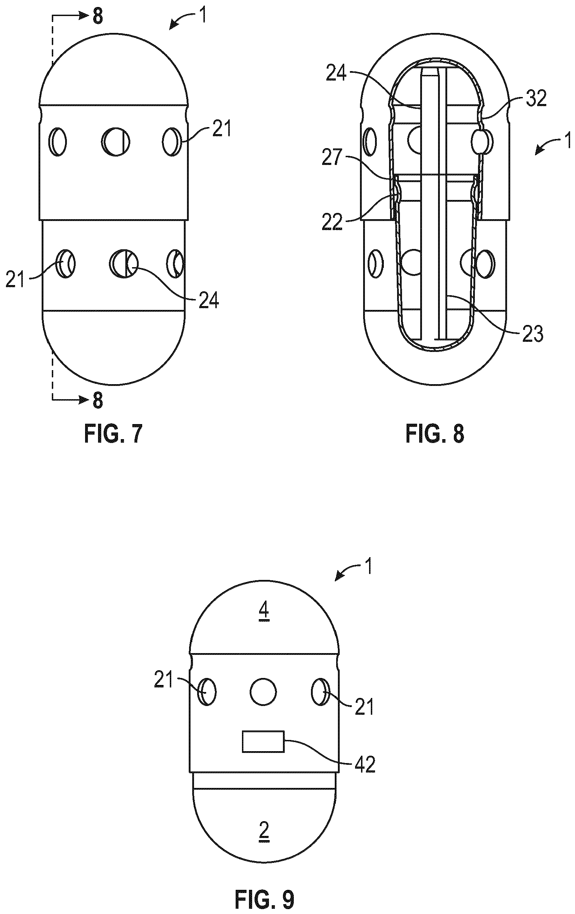

**FIG. 7** is a side view of the inner casing **2** partially and slidably inserted into the outer casing **4** and thereby assembled into the gastrointestinal sampling device **1** in its open configuration. As can be seen ports **21** are all open and strut **24** is in place keeping the device in its open configuration.

**FIG. 8** presents a cut away view of **FIG. 7** revealing both the strut **24** supporting the open port configuration and elastomer filament **23** providing a persistent contracting force in contravention. **FIG. 8** also shows the female stop ring **22** and the male stop ring **32.** When the strut **24** fails and the elastomeric filament **23** pulls the inner and outer casings **(2, 4)** together, the male stop ring **32** inserts itself into the female stop ring **22**, thereby encouraging the gastrointestinal sampling device **1** to stay in its closed configuration along with any residual tension from the elastomeric filament **23**. It should be understood that in other equivalent embodiments the stop rings **22** and **32** may be designed differently as may be known in the art with a similar effect. For example, there may be a stop ring, or rib, or edge created on the exterior side of wall **27** that engages and stops the sliding advance of the edge of outer casing **4**. Hence, the example of stop rings **(22, 32)** described herein is a non-limiting example.

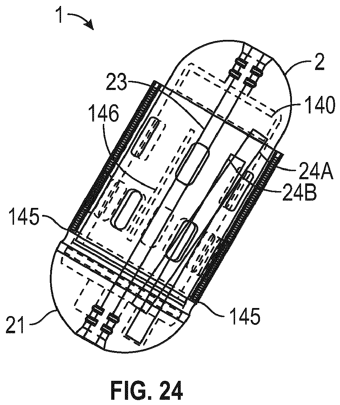

**FIG. 9** is a side view of the gastrointestinal sampling device **1** in its closed configuration. As a means of identifying one gastrointestinal sampling device **1** from another, each device may carry a radio frequency identification device (RFID) transponder **42.** A RFID transponder in its various configurations and uses, per se, is well known in the art and need not be discussed further herein. It should be noted that in some embodiments, when the strut **24** collapses, some mechanical work is done as the casings pull together. Devices may be incorporated into the casings to convert that work to electrical energy for a variety of uses such as active RFID communications to indicate outside the body when a capsule enters its closed configuration. An exemplary device including a linear motion power generator is described in U.S. Patent 5,347,186 to Konotchick, which is included herein by reference in its entirety. Hence, the outer casing may include an embedded coil Q and the inner casing may include a companion set of one or more linear conductors or magnets R, or vice versa (See, e.g., **FIG. 24**). The coil should be counter sunk and encased to avoid a short circuit from direct electrical contact with the magnet or the sample fluid. The coil may be comprised of a non-ferrous material so that the magnets do not interfere with the collapsing of the capsule to its closed configuration.

**FIG. 10** is a side view of another embodiment of the gastrointestinal sampling device **1**. The gastrointestinal sampling device **1** is similar to that of combined **FIGs. 1-2**. However, the gastrointestinal sampling device **1** of **FIG. 10** has one receiver **29**, which is in outer casing **4** and comprises only three channels **20**. The receiver **25** in inner casing **2** has no channels. In this embodiment, the strut **24** merely abuts the flat surface of the receiver **25.** The strut **24** of the embodiment of **FIG. 10** is a compound strut comprising two or more hydrophobic components **24A** and **24B** secured together by a hydrophilic attachment means **33.** A non-limiting example of a hydrophilic attachment means is a polyvinyl alcohol pin, a polyvinyl alcohol band, or polyvinyl alcohol glue.

In other equivalent embodiments the gastrointestinal sampling device **1** may include a pliable or an elastomeric gasket **140** penetrated with a hole. For example, **FIG. 20** illustrates a non-limiting example of such a gasket. The exact size and shape if gasket **140** may vary according to the shape of the gastrointestinal sampling device **1** and as may be expedient in manufacturing, including filling the entire end portion of the outer capsule **4** and may extend into the cylindrical portion of the outer capsule **4**. The purpose of the gasket is to provide a fluid tight or at least a fluid resistant seal around the edge of the inner capsule **2** to prevent fluid leakage or communication after the strut **24** has collapsed and the gastrointestinal sampling device **1** has closed.

In still other equivalent embodiments, the gastrointestinal sampling device **1** may include a sponge, absorbent material, or super absorbent material **142.** The absorbent material may be a dried or pre-compressed compressed strip or stick of material that is either secured or unsecured to the inside gastrointestinal device **1**. In other embodiments the dry absorbent material may circumferentially line the interior circumference of the inner capsule **2**, keeping in mind that too much absorbent material may prevent the gastrointestinal sampling device **1** from closing as it expands with fluid and fills the interior of the device. A non-limiting example of absorbent material may be a non-dissolving polyvinyl alcohol foam such as that produced by Ramerfoam Technology from Honiton, Devon, Great Britain or a compressed cellulose like Spontex made by Newell Brands.

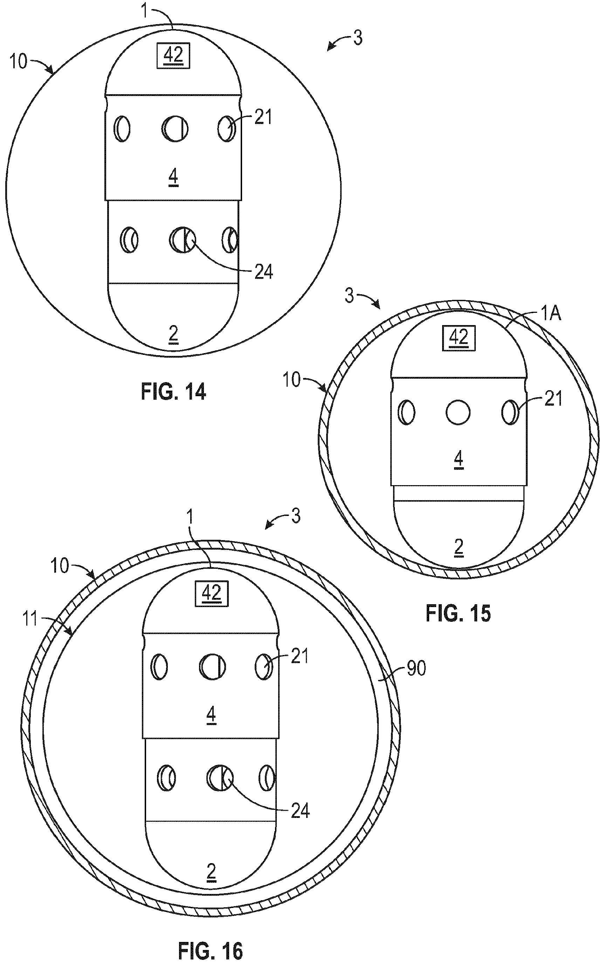

In use as a sampling system **3** (**see,** **FIGs. 14-15**), the gastrointestinal sampling device **1** is fluidly isolated within one of a wide choice of available delayed or immediate release digestible (e.g., "enteric") outer capsule(s) **10** (**see,** **FIG. 14**) as are known in the art, and is ingested. The outer capsule **10,** or parts thereof, may be chosen or designed to dissolve in a proscribed amount of time after ingestion, which implies failure of its fluid tight integrity at an approximate location along gut length x. Design parameters of the outer capsule related to time of failure of its fluid tight integrity include choice of material, thickness of the material, shape of the capsule, and mechanical weakening to intentionally create weak points in the capsule that may fail faster than a general failure. Because stomach acid ranges from a pH of 1-3.5, the outer capsule **10** may necessarily need to be acid resistant below a 3.5 pH from a few minutes for liquid stomach contents to five hours for stomach contents with meat and high fat substances. Thickness and material can adjust for this timing.

The digestible outer capsule **10** is an excipient that dissolves in the stomach or intestines in a desired timeframe thereby providing fluid access to and through the normally open ports **21** of the gastrointestinal sampling device **1.** The fluid flow comprises local community microbiota along with their environment including ingesta, loosely adherent mucus layer particles, and bodily chemistries into the gastrointestinal sampling device **1**. The fluid flow enters the gastrointestinal sampling device **1**, envelopes the dissolvable, non-toxic strut **24** causing it to fail structurally, which in turn allows the tension provided by the elastomeric filament **23** to pull or slide the inner casing **2** further into the outer casing **4** and hold gastrointestinal sampling device **1** in a closed, sealed port position (**see,** **FIG. 13**). The capsule continues through the gastrointestinal tract and is recovered from the stool, cleaned, identified by the RFID tag **42**, and can be opened for analysis or frozen for future analysis. The RFID tag contains information such as when the gastrointestinal sampling device was ingested, in what order, and the construction parameters of its digestible outer capsule **10,** as non-limiting informational examples. The circuitry of the RFID tag may include a sub-circuit wherein the sub-circuit is closed when the inner capsule **1** is in the closed configuration and is open in the open configuration. As such, it can be electronically determined where the capsule is located and if it is open or closed. A device with multiple receivers or multiple transceivers may provide a location in three dimensions.

As discussed above the physical and chemical parameters of the digestible outer capsule **10** will indicate approximately where in the gastrointestinal tract the sample was taken based on an expected elapsed time to dissolution. Alternatively, the outer capsule **10** may be comprised of a material that only deteriorates in the presence of certain pH, enzymes, bacteria, or the immediate byproducts thereof.

The outer digestible capsule(s) **10** may be a standard state of the art, publicly available or proprietary, biomedical immediate or delayed release gastrointestinal capsule. One or more enteric capsules are capable of protecting the inner capsule from the strong acidity of the stomach for later disintegration in the intestines. Other non-limiting examples of possible digestible materials include polyvinyl alcohol, seaweed derivatives from the Evoware Company of Indonesia, casein proteins developed by the US Department of Agriculture, Agar, and the Cassava root from the Avani Company of Bali. Other non-limiting examples of biodigestible polymers may include Polyactic acid and Polly-3-hydroxybutyrate (PHB). Suitable proprietary materials may also be obtained from Capsugel of Morristown, Jew Jersey, USA and the Eudragit^{®} brand of pH sensitive materials from Evonik Industries AG of Essen, Germany.

In short, any suitable medical grade dissolvable/digestible material may be used to produce the outer capsule(s) that currently exists or that may be developed in the future. The outer capsule may be approximately 25.5mm in length with a diameter of approximately 8.7-9.95 mm.

In other embodiments, an interstitial capsule or "skin" **11** (**see,** **FIG. 16**) may be incorporated between the outer capsule **10** and the gastrointestinal sampling device **1**, thereby providing a two-parameter selection mechanism for taking a sample. For example, the outer capsule **10** may fail at a pH of 5.8 or less and the inner thin capsule **11** may be designed to deteriorate on contact with a certain bacterium. Only when both capsules fail will a sample be captured as discussed above.

In some embodiments, the outer capsule **10** and the interstitial capsule **11** are double ended, two-part hard capsules (or half-capsules) of similar size and shape to the gastrointestinal sampling device **1**, but without any ports **21**. The interstitial capsule **11** may be manufactured to encapsulate and protect the gastrointestinal sampling device **1.** The outer capsule **10** may be manufactured to encapsulate the interstitial capsule **11** and a delivery payload placed between at least a portion of the interstitial capsule **11** and the outer capsule **10** such that when the outer capsule dissolves the payload is released, which could be a drug, medicine or nanostructures/machines. Hence, it is contemplated herein that composition and structure of the outer capsule **10**, the inner capsule **11**, and hydrophilic strut **24**, of the gastrointestinal sampling device 1 may be designed to fail in a specific sequence, timing, and/or a series of locations in the digestive tract.

RFID devices or circuits engineered into one or more of the nested capsules may be designed to respond while each capsule retains its original physical fluid tight integrity but cease to operate when its capsule loses its original physical fluid tight integrity. As such, device performance and its general location along the track can be monitored in real time.

In some additional embodiments the outer half-capsule (not shown) of the outer capsule **10** comprises a first dissolvable material and may be secured fluid tight over at least part of the inner half-capsule (not shown) of the outer capsule **10** that comprises a different second dissolvable material.

The inner half-capsule (not shown) of the outer capsule **10** may also be adhered fluid tight to part of the outside of the interstitial capsule **11**. A designed space between the interstitial capsule **11** and the inner half-capsule of the outer capsule **10** may contain a payload comprising a medicine, a drug, or a number of nanostructures that are thereby protected from digestive fluids for a desired travel time to a particular location. The rounded outer half capsule (not shown) of the outer capsule **10** may be comprised of a fast-dissolving substance such as gelatin that falls away in or just after exiting the stomach and be desired for ease of swallowing. The inner half-capsule of the outer capsule **10** is of such a material and thickness to provide the desired travel time for the payload.

Further, the gastrointestinal sampling system **3** may include other embodiments of ingestible capsule **10** that may be used independently or in conjunction with the ingestible capsule **1A** of **FIG. 15**. For example, **FIG. 15** depicts another embodiment of the gastrointestinal sampling device **1A**, that may operate to deliver a drug, device or other substance to a targeted area of the digestive tract. In this embodiment, the strut **24** and elastomeric filament **23** of **FIG. 1** both may be replaced with a compressed spring **124**. Or alternatively, the strut **24** may be shortened and a compressed spring **124** added to the end being inserted into the outer casing **2.** The casings **(2, 4)** may then be filled with a medicine or drug and then the casings compressed together and inserted into a digestible outer capsule **10** of such dimensions so as to keep the gastrointestinal delivery device **1A** in its closed configuration. The outer capsule **10** may be constructed of such a material and construction that the patient's digestion causes the outer capsule **10** to fail at an expected location in the digestive track, thereby releasing the payload substance as the spring **15** forces the gastrointestinal delivery device **1A** into its open configuration. Non-limiting exemplary payloads may be any of medicines, chemical substances, enzymes, antibiotics, proteins, fecal matter, fecal transplants, nanostructures, nano-robots, and microbes.

An exemplary technique using a coordinated set of gastrointestinal sampling devices **1** and **1A** may include two separate sampling devices **1** and one delivery device **1A** being ingested at the same time with slightly different-to dissolution time settings built into the material of the outer capsules **10**. The three devices should travel relatively close together through the digestive tract. Timing differences may be constructed into each capsule such that a first sampling device **1** triggers into its closed configuration capturing a "before" sample. A specified time later the delivery device **1A** triggers into its open configuration to release a medicine. And, at a third time the second sampling device **1** triggers into its closed configuration to take a second "after" sample of the material that should still be in the proximity of the first sample.

In still further embodiments, the sampling capsule system **1** disclosed herein may be used in conjunction with nano-objects, such as a nano-box with or without an articulating lid, to more efficiently deliver substances and collect samples. A non-limiting example of a nano-object is a DNA/RNA Origami object that is more fully described in U.S. patents 9,765,341 issued September 9, 2017 to Bachelet and 9,944,923 issued April 17, 2018 to Blattman, each of which is herein incorporated by reference in its entirety. Amongst other nanomaterials, a preferable material for a nanostructure is iron.

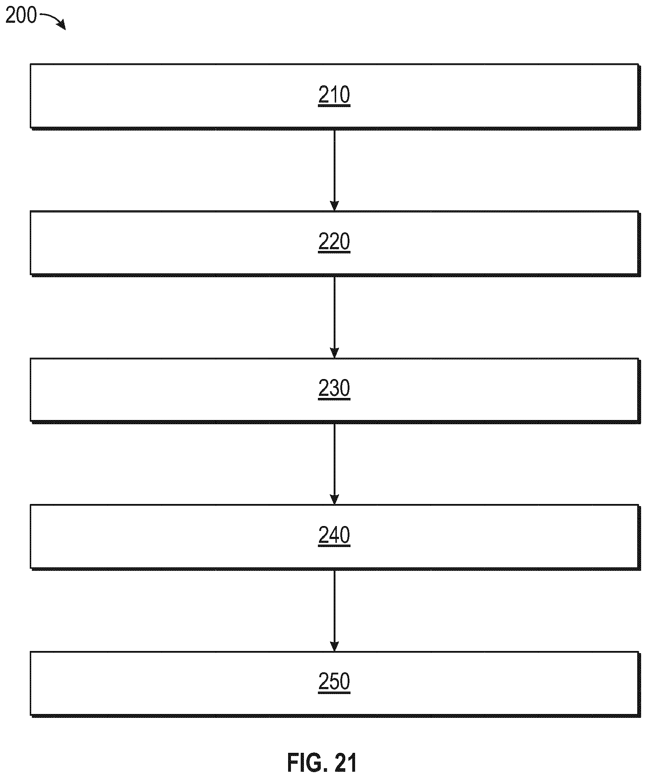

**FIG. 21** is an exemplary logic flow chart illustrating a method **200** of operation of the three capsule sampling system **3** based on a single exemplary embodiment of the capsule. Those skilled in the art will find modifications of the structure of the capsule useful to meet specific operational requirements. Hence, **FIG. 21** is merely illustrative and is not intended to limit the scope of the inventions disclosed herein.

At process **210**, the assembled capsule sampling system **3** is ingested. In some embodiments the capsule sampling system **3** may be configured to have an interstitial space **90 (See e.g.,** **FIG. 16****)** between the outer capsule **10** and the interstitial capsule **11**. Outer capsule **10** and interstitial capsule **11** may be comprised of a double ended, two-part, oblong capsule without any ports in a similar oblong shape of a sampling capsule **1**. Such an interstitial space **90** may be located in one end of the oblong shaped outer capsule **10** although such a location is not intended to be limiting. Selecting a specific half capsule having a longer specific length relative to the corresponding half capsule of an oblong interstitial capsule **11** is one way of controlling the existence and size of the interstitial space.

The two half capsules of the outer capsule **10** may be designed to dissolve in different pH liquids. In this example, the half-outer-capsule that does not contain the drug payload may have a pH tolerance of 6.5 or higher and therefor dissolves in the acidic stomach fluids (pH 1.0-3.5) almost immediately at process **220** and exposing only half of the interstitial capsule **11**.

The second half-outer-capsule that contains the drug payload may have a pH sensitivity of 6 or higher and is adhered fluid tight to the outside surface of the interstitial capsule **11,** thereby fluidly isolating the drug payload in the interstitial space. As such, the second half outer capsule does not dissolve in the acidic stomach fluid but is passed into the intestines (pH 6-7.4) along with the intact drug payload at process **230**. The pH of the intestines is known to vary from location to another along the track. As such, the pH sensitivity of the second half-outer-capsule may be selected so that the second half-outer-capsule dissolves at a specific pH in a targeted location and releases the drug payload at process **240**. It should be noted that instead of gluing, other means of fluid tightly attaching the inside of the outer capsule to the outside of a more inner capsule may include a friction fit circumferential male/female collar arrangement that may or may not include an O-ring.

The intact interstitial capsule **11** travels through the intestines having a different pH sensitivity than the second half capsule of the outer capsule. At process **250**, the interstitial capsule dissolves at the targeted location based on local pH, thereby allowing local fluids to enter the open ports **21** of the sampling capsule **1.** The sampling capsule **1**, then shuts when the hydrophilic strut **24** dissolves and collapses, thereby capturing and fixing a fluid sample as further described herein.

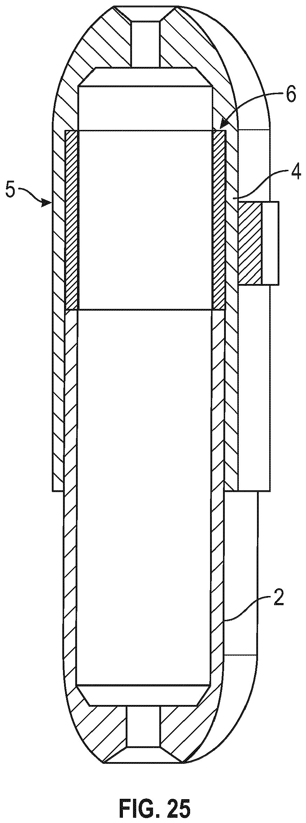

**FIG. 25** is an equivalent alternative embodiment of the sampling capsule using a thin lining, insert or collar **6** that replaces the hydrophilic strut **24.** The collar **5** encircles or lines the inner surface of the outer casing **4.** The insert **5** is tight fit/pressure fit or glued with hydrophilic adhesive to the outer casing. The inner casing **2**, under tension provided by the hydrophobic elastomeric filament **23** (not shown), rides a first annular edge **6** of insert **5**. The outer casing has an annular lip/rim/shelf that engages the opposite annular edge **7** (see FIG. 1) of insert **5.** Thus, insert **5** holds the sample capsule in its open configuration in place of strut **24**. When the hydrophilic insert **5** dissolves from fluid contact or pH exposure over a pre-determined and engineered time period, the elastomeric filament **23** (not shown) pulls the inner and out casings together into the capsule's closed configuration such that the inner casing ledge **6** (See FIG. 2) seats itself against the annular lip/rim/shelf **7** formed inside of the outer casing. It should be understood by those of ordinary skill in the art that if the insert **5** is attached to the inner surface of the outer casing **4,** or patch glued to the edges of the ports **23,** by an adhesive with strong enough adhesive strength, the collar length may be shortened to produce essentially an annular ring (e.g., an O-ring). Adhesion is described by tensile adhesion strength or shear adhesion strength and is expressed in terms of N/mm² or Megapascals (MPa). This ring alternative insert embodiment reduces the cost of the collar material per sample capsule **1** and minimizes the amount of material on the inner surface of the outer casing that may jam the collapsing action and prevent the ports **21** from becoming sealed in the closed configuration.

In another alternative embodiment, collar **5** may be configured to fully prop the inner and outer casing apart as shown in **FIG. 25**, but to also protrude outward through ports **21** and plugging (e.g., sealing) the ports fluid tight. The thickness and/or chemical makeup of various parts of this collar embodiment may be engineered by one of ordinary skill in the art to dissolve through at the ports **21** first, allowing a sample to be captured before the sample capsule contracts to its closed configuration when the rest of the collar or ring gives way, capturing a sample. Hence, an outer enteric delivery capsule **10**, may be reduced in thickness or changed in chemical makeup, or eliminated entirely.

In yet another alternative embodiment, the insert/collar **5** may be replaced with dots, bumps or other geometric protrusions (not shown) of hydrophilic, dissolvable materials disclosed here in or that may be within the knowledge of one of ordinary skill in the art at calculated positions on the inside surface of the outer casing **4** to keep the capsule in its open configuration. These bumps or protrusions may look similar to spot welds spaced around the inside perimeter of the Outer Casing **4**. The size and geometry of the protrusions or bumps may be chosen to adjust their time-to-failure and/or to minimize interference with the capsule sliding into its closed configuration due to retrograde amounts of hydrophilic material. In some embodiments the protrusion or bump may be manifested as a thin bump with the same thickness as the outer casing **4** that would be configured/placed to hold against the pulling force of the elastomeric filament. The thin bump should not be thicker than the casing and should include enough clearance space to allow it to be inserted into an outer enteric delivery capsule **10.**

When the protrusions dissolve, the Inner Casing **2** slides to the Outer Casing stop, seat or ledge **7,** thereby covering the port(s) **21** and sealing the sample inside the capsule. The protrusions provide for control over how long they hold against the tension from the elastomeric filament. There may be any number protrusions (equally spaced or otherwise) with the number preferred to be from one to four. Non-limiting examples of these protrusions may be approximately .5mm in height and less than 1mm in circumference/perimeter.

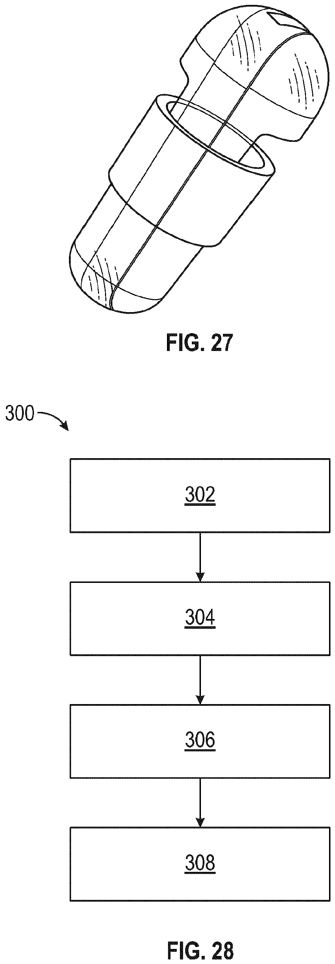

There are multiple conceivable methods to manufacture the gastrointestinal sampling device **1.** A preferred method **300** for at least one embodiment shown in **FIG. 28** is to manufacture the casings inside out and roll them into their proper configuration. At process **302** the sampling capsule is molded as an inverted molded one-piece part made of stretchable silicone rubber such as Elastostar ESR 402/40 having a Shore A value of 42. This inverted configuration is the same configuration as disclosed in FIGs. 8-9 of the co-owned provisional applications 62/573,587 filed in December 2017 and 62/682,091 filed in June 2018. At Process **304,** the inner and outer capsules are pulled apart thereby placing a strain on the elastomeric filament. At process **306**, a strut 24 is inserted into receptacles the closed ends of each casing **2** and **4,** thereby maintaining the filament in a strained, open condition. At process **308,** the open ends of each casing are rolled down their outside surfaces inside out such that the outer casing overlaps the outer surface of the inner casing, leaving the capsule in its open configuration.

The capsule **1** in the newly molded bar bell configuration has an elastomeric, slidable Inner Casing **4** opposing an elastomeric, slidable Outer Casing **2** connected together with an elastomeric filament **23** extending between the casings, continuing through the casings and for the full extent of the casings lengths as shown in **FIG. 27**. Assembly of the capsule at process **204** consists of inverting (i.e., rolling down or rolling inside out) the stretchable casings and guiding the inner casing into the outer casing. At process **206** the casings are stretched apart and at process **208** a hydrophilic strut 24 is mounted between the opposing strut mounting holes.

Alternatively, the casings **(2, 4)** may be individually molded and an elastomeric filament **23** adhered or glued into the inside ends of the casings, or to indentations in the inside ends or holes in the inside ends. In some embodiments the glue does need to adhere the filament into a hole or indentation in the end of the casing but may merely cure in place thereby mechanically preventing the filament from pulling out or away from the casing under tension.

After rolling right side out, the casings are released leaving the casings **(2,4)** overlapped and the capsule **1** in an open position. The exposed filament **23** that was extending through the casings is trimmed. When the open capsule **1** is exposed to fluids flowing through ports **21**, the fluids dissolve and weaken the strut allowing the cord to pull the sliding casings closed, covering the ports, and sealing in the fluid sample.

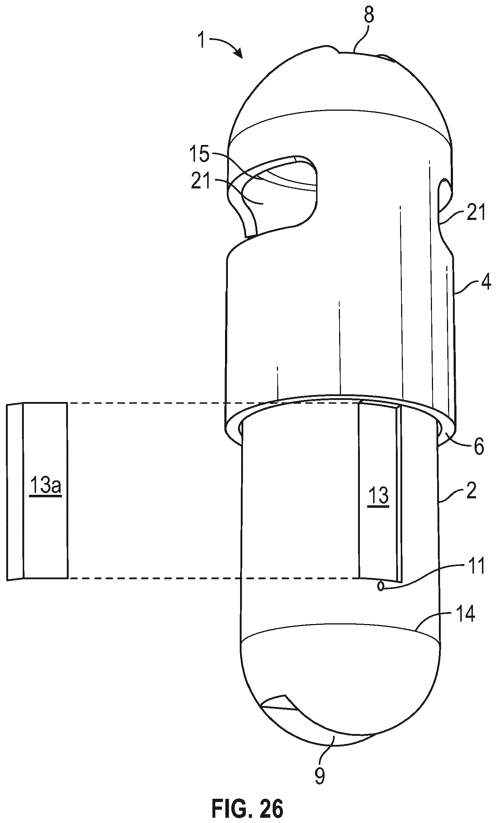

**FIG. 26** illustrates another equivalent embodiment of the capsule where internal hydrophilic strut **24** used in other embodiments to hold inner and outer casings (**2, 4**) apart is replaced with an external insert **13a** which may be dissolvable or not. Insert **13a** is depicted herein as a solid cuboid. However, insert **13a** may be formed in any shape as may be convenient or advantageous, such as a cube, a cylinder, dome, or a pyramid and may be hollow or solid. Insert **13a** is attached to the outer surface of the inner casing **2** for the purpose of holding the capsule **1** in its open configuration by interfering with the outer capsule's ability to slide over the inner casing. Insert **13a** may be glued to the smooth outer surface of the inner casing directly, or it may be adhered in a cutout **13.** Like strut **24** the insert **13a** may be comprised of any dissolvable material formed in any shape or thickness in order to efficiently act as a timing device that enables the elastomeric filament to draw the capsule **1** closed fluid tight. The potential combinations and permutations of materials, shapes and sizes are innumerable and cannot be discussed herein in any meaningful detail. However, any combination is contemplated herein as functional equivalents.

Using a cuboid as an example, as shown the cuboid would be formed with a thickness that exceeds the height of the cutout **13** so that the top end as shown rises above the surface of the inner casing **2** and engages the open end of the outer casing **4**, thereby maintaining the capsule in its open configuration against the closing force of the elastomeric filament. As the exposed outer surfaces of the insert dissolve, the rise above the inner casing surface decreases until such time as the outer casing **4** can be forced over the cutout **13** and place the capsule in its closed configuration. A cuboid would have a different dissolving time as opposed to an upright pyramid, for example.

In embodiments where the insert **13a** is not hydrophilic (i.e., dissolvable), cutout **13** should be larger than the insert **13a** so that the hydrophilic glue adhering it in place is exposed to the gastrointestinal fluids and dissolves in a predetermined, predictable fashion. In this non-limiting exemplary embodiment, if the insert **13**a is not dissolvable and is adhered to the smooth outer surface of the inner casing, it will simply be pushed aside by the outer casing under the elastomeric filament tension as the hydrophilic glue weakens. In some embodiments with an external strut, the external strut may prop the first casing away from the second casing against the strain of the elastomeric loop by abutting the open end of the casing having the larger diameter and abutting a buttress formed on the casing having the smaller diameter. The buttress may be a dollop of a dissolvable/hydrophilic adhesive, for example. An equivalent example of a buttress may be a hydrophobic element adhered to the smaller casing by a hydrophilic adhesive.

If the insert **13a** is adhered into a cutout **13**, the cutout should be of such dimensions as to be wider than those of the insert **13a** to allow gastrointestinal fluids to immediately contact and begin dissolving the hydrophilic adhesive. Further, to facilitate the non-dissolving insert **13** falling out and away from the cutout **13** at the pre-determined time, the distal end or distal perimeter portion of the cutout **13**, may be removed during manufacture to allow the insert to freely slide out of the insert.

In the embodiment of **FIG. 26**, the elastomeric filament **23** may be manifested as a loop of elastomeric material in a ring or oval shape like a rubber band (See, **FIG. 27**). Instead of being used inside capsule **1**, the filament band **23** here is looped longitudinally around the exterior of capsule **1** that is held in the open configuration by insert **13a** or an interior strut **24**. The external band **23** inserted or secured in cutouts **8** and **9** formed in the external surfaces of the closed ends of both the inner and outer casings as shown in **FIGs. 26** and **27****.** The band 23 may be secured by lips or overhangs created in the casing(s) (Not shown).

It should be noted that in some embodiments, the outer casing **4** may have an inner shoulder **15** formed along its inner circumference and the inner casing **2** may have an outer shoulder **14** formed around its outer circumference. Each shoulder may include an O-ring (not shown), to enhance its fluid tightness when in its closed configuration.

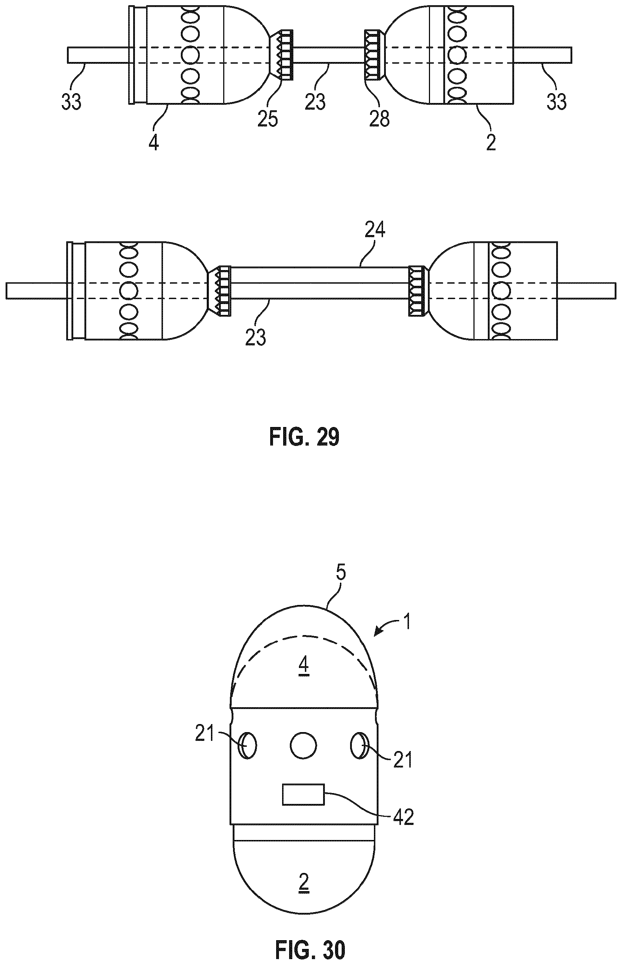

In yet another embodiment (see **FIG. 30**), the assembled capsule **1** may include a third casing **5** (**a "piggy back" casing**) that is attached to the outer surface(s) of the inner and outer casings **2** and **4.** This third casing may configured to create a liquid tight volume between its inner surface and the outer surfaces of casing **2** and/or **4** that may be used to contain a payload such as medication, nanoparticles, drug or marking substance as non-limiting examples. This third casing **5** may be non-dissolvable or hydrophobic and be attached to one or both inner and outer casings with a time delayed dissolvable adhesive. When the adhesive weakens and gives way at a predefined time, the piggyback casing falls away releasing its payload. The timing of the strut or insert release/collapse may be coordinated with the piggyback capsule jettison so that the sample collected by capsule **1** is one that reflects the effect of the payload on the fluid sample captured, or not. The piggyback capsule may be configured to cover one or more ports **21.**

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment of the invention. It being understood that various changes may be made in the function and arrangement of elements described in an exemplary embodiment without departing from the scope of the invention as set forth in the appended claims.

## Claims

1. A sampling capsule, comprising:
a ported first casing with an open end, a closed end, a first diameter, an inner surface, and an outer surface,
a second casing with an open end, a closed end, a second diameter, an inner surface, and an outer surface, wherein the outer surface of the open end of one of the first casing and the second casing slidably engages the inner surface of the open end of the other casing permitting a sliding transition of the capsule from an open configuration to a fluid tight, closed configuration,
wherein one of the first diameter and the second diameter is larger than the other.

2. The sampling capsule of claim 1 further comprising an elastomeric loop having a length, wherein the filament fixedly secures the closed end of the first casing to the closed end of the second casing.

3. The sampling capsule of claim 2, wherein the elastomeric loop encircles the exterior of the sampling capsule when the sampling capsule is in its open configuration and in its closed configuration.

4. The sampling capsule of claim 3 further comprising a hydrophobic strut configured to space the closed end of the first casing away from the closed end of the second casing and imparting a first tension to the elastomeric loop.

5. The sampling capsule of claim 4, wherein the hydrophobic strut is located on the exterior of the casing with the smaller of the first diameter and the second diameter.

6. The sampling capsule of claim 5, wherein one of the first casing and the second casing is configured to slidably insert into the other casing.

7. The sampling capsule of claim 6, wherein the elastomeric loop has a length that is configured to have a first tension when the capsule is in an open configuration and a second, lesser tension when the capsule is in a closed configuration.

8. The sampling capsule of claim 7, wherein the hydrophobic strut abuts the open end of the casing with the larger diameter and abuts a buttress comprising a dissolvable material.

9. A method for passive sampling of a bodily fluid in a patient, the method comprising:
administering to a patient an ingestible device comprising:
a capsule housing that bounds a hollow collection cavity,
a sampling aperture formed in the capsule housing configured to provide fluid communication between the collection cavity and the bodily fluid exterior to the capsule housing,
a collection cavity sealing means;
mechanically sealing the sampling cavity by the sealing means; and
collecting the mechanically sealed ingestible device from the stool of the patient.

10. The method of claim 9, where the capsule housing comprises:
a ported first casing with an open end, a closed end, a first diameter, an inner surface, and an outer surface,
a second casing with an open end, a closed end, a second diameter, an inner surface, and an outer surface, wherein the outer surface of the open end of one of the first casing and the second casing slidably engages the inner surface of the open end of the other casing permitting a sliding transition of the capsule from an open configuration to a fluid tight, closed configuration, wherein one of the first diameter and the second diameter is larger than the other.

11. The method of claim 10, wherein the capsule housing includes a hydrophobic strut.

12. The method of claim 11, wherein the capsule housing includes an elastomeric loop connecting the ported first casing to the second casing on the exterior of the capsule housing.

13. The method of claim 11, wherein the capsule housing includes an elastomeric filament connecting the ported first casing to the second casing on the inside of the capsule housing.

14. The method of claim 10, wherein a dissolvable material over the sampling aperture is configured to dissolvable open the sampling aperture.

15. A method of manufacturing a device for passive sampling of a gastrointestinal tract, the method comprising:
providing a capsule housing having a cavity formed from a first casing with a first diameter and an exterior surface and a second casing with a second diameter and an exterior surface and at least one sampling aperture penetrating through one of the first casing and the second casings, the sampling aperture providing fluid communication between the cavity and an exterior of the capsule housing;
adhering a buttress to the exterior of the casing with the first diameter;
inserting a strut with a first end and an opposing second end onto the exterior of the casing with the first diameter, wherein the first end of the strut abuts the buttress and the second end of the strut abuts the casing with the second diameter, and
attaching an exterior elastomeric loop that secures together the casing with the first diameter and the casing with the second diameter, wherein the strut opposes a closing tension imparted by the elastomeric loop.
